# EUROPÄISCHE PATENTSCHRIFT

(11) **EP 0 736 315 B1**
(45) Veröffentlichungstag und Bekanntmachung des Hinweises auf die Patenterteilung: **29.08.2001**
(21) Anmeldenummer: 96104473.2
(22) Anmeldetag: 21.03.1996
(51) Int. Cl.: B01D 7/02

(54) **Kristallisator**
Crystallizer
Cristallisoir

(30) Priorität: 06.04.1995 DE 19512845
(43) Veröffentlichungstag der Anmeldung: 09.10.1996
(73) Patentinhaber: Balcke-Dürr Prozesstechnik GmbH, 40882 Ratingen (DE)
(72) Erfinder: Kassat, Harry, 44797 Bochum (DE)
(74) Vertreter: Stenger, Watzke & Ring Patentanwälte

(56) Entgegenhaltungen:
- EP-A- 0 285 504
- DE-B- 1 072 965
- US-A- 3 609 943
- PATENT ABSTRACTS OF JAPAN vol. 012, no. 120 (M-685), 14.April 1988 & JP-A-62 245085 (TOSHIBA CORP), 26.Oktober 1987,
- PATENT ABSTRACTS OF JAPAN vol. 007, no. 081 (M-205), 5.April 1983 & JP-A-58 008997 (TOKYO SHIBAURA DENKI KK), 19.Januar 1983,
- PATENT ABSTRACTS OF JAPAN vol. 010, no. 070 (M-462), 19.März 1986 & JP-A-60 213796 (HIRAKAWA TEKKOSHO:KK), 26.Oktober 1985,

## Beschreibung

Die Erfindung betrifft einen Kristallisator zur Gewinnung von sublimationsfähigen Produkten aus der Gasphase gemäß den Merkmalen im Oberbegriff des Anspruches 1.

Zur Gewinnung von sublimationsfähigen Produkten aus der Gasphase, insbesondere von Phthalsäureanhydrid (PSA) aus Reaktionsgasen, ist es bekannt, das Reaktionsgas quer zu in einem Behälter angeordneten Rippenrohren zu führen, die innenseitig mit einem Kühlmedium beaufschlagt sind. Durch den Kühlprozeß schlägt sich das Produkt außenseitig an den Rippenrohren nieder. Diese Maßnahme kann so lange durchgeführt werden, bis die Rippenrohre eine Beladung aufweisen, daß die Gasströmung quer zu den Rippenrohren merklich behindert wird. Nunmehr wird die Zuführung des Reaktionsgases zum Behälter abgebrochen. Anschließend werden die Rippenrohre innenseitig mit einem Heizmedium beaufschlagt, wodurch das Produkt von den Rippenrohren abgeschmolzen wird und sich bodenseitig des Behälters sammelt. Von hier aus wird das Produkt abgezogen und der weiteren Behandlung zugeführt.

Aufgrund der Diskontinuität der Beladung der Rippenrohre und ihrer Abschmelzung werden in der Praxis mehrere Kristallisatoren so zeitlich differenziert aufeinander abgestimmt beladen und abgeschmolzen, daß nahezu kontinuierlich ein gleichmäßiger Produktanfall gewährleistet werden kann.

Ein für diesen Zweck bekannter Kristallisator besteht aus einem Behälter mit einer oberen gewölbten Gaseintrittshaube, aus Rippenrohrbündeln im umfangsseitig durch Seitenwände und Stirnwände begrenzten mittleren Zentralbereich und einer unteren Produktsammelwanne. Jedes der übereinander angeordneten Bündel umfaßt nebeneinander liegende Rippenrohre langgestreckter U-förmiger Konfiguration. Die geraden Schenkel der einen ovalen Strömungsquerschnitt aufweisenden Rippenrohre erstrecken sich horizontal und liegen übereinander. Jeweils einander benachbarte Rippenrohre sind in der Höhe zueinander versetzt und auf Lücke angeordnet. Bei den Rippen handelt es sich um rechteckige Einzelrippen, die umfangsseitig der Kernrohre festgelegt sind.

Die Schenkel jedes Rippenrohrs sind an einem Ende durch unberippte im Behälter liegende Rohrbögen mediumleitend miteinander verbunden. Die Rohrbögen werden an die Schenkel geschweißt. Die freien Schenkelenden der Rippenrohre jedes Bündels sind in eine Stirnwand des Behälters eingeschweißt und münden in stirnseitige Verteiler- bzw. Sammelkammern für das Kühlmedium und das Heizmedium. Dabei sind die Verteiler- und Sammelkammern von Bündel zu Bündel einmal an der einen Stirnwand und einmal an der anderen Stirnwand des Behälters vorgesehen. Hiermit wollte man erreichen, daß der im Bereich der unberippten Rohrbögen nur unzureichend gekühlte Gasstrom anschließend durch einen berippten Bereich mit intensiver Kühlung geführt wird. Dadurch ist es z.B. bei vier übereinander angeordneten Bündeln erforderlich, nicht nur an beiden Stirnwänden des Behälters Zuführleitungen und Abführleitungen für das Kühlmedium und das Heizmedium vorzusehen, sondern diese auch noch an jeder Stirnwand zu verzweigen. Der Aufwand für die Verlegung dieser Leitungen zu den Verteiler- und Sammelkammern ist mithin beträchtlich.

Die unberippten Rohrbögen der Rippenrohre liegen in ausreichendem Abstand von den Innenflächen der Stirnwände. Diese Maßnahme ist erforderlich, damit die Rippenrohre sich ausreichend axial dehnen und wieder kürzen können.

Der bekannte Kristallisator wird bei der Herstellung stufenweise von unten nach oben aufgebaut. Zunächst wird unter Einsatz von Hilfseinbauten das unterste Bündel aus den Rippenrohren zusammengestellt. Anschließend werden die freien Schenkelenden dieser Rippenrohre mit einem Stirnwandabschnitt verschweißt. Außerdem wird das Bündel auf einer Tragkonstruktion abgestützt, die sich im Behälter von einer Seitenwand zur anderen erstreckt.

Letztlich werden im Höhenbereich des Bündels die Stirnwandabschnitte mit den angrenzenden Seitenwandabschnitten verschweißt. Sind das unterste Bündel sowie der Behälterabschnitt derart ordnungsgemäß lagefixiert, wird in derselben Art und Weise das nächste Bündel bezüglich der einzelnen Rippenrohre aufgebaut und mit den in diesem Höhenbereich befindlichen Stirnwandabschnitten und diese mit den angrenzenden Seitenwandabschnitten verschweißt. Ferner werden diese Behälterwandabschnitte mit den darunter liegenden Wandabschnitten verschweißt.

Sind sämtliche Bündel und alle Behälterwandabschnitte übereinander lagefixiert, werden diese auf die Produktsammelwanne mit der Tragkonstruktion gesetzt und mit dieser verschweißt. Schließlich wird der Kristallisator durch Aufsetzen der Gaseintrittshaube und Verschweißen mit derselben mit den angrenzenden Stirnwand- und Seitenwandabschnitten umfangsseitig des obersten Bündels verschweißt.

Der für den Zusammenbau des bekannten Kristallisators erforderliche Aufwand ist mithin beträchtlich.

Neben dem voranstehend beschriebenen, aus der Praxis bekannten Kristallisatortyp sind ähnlich aufgebaute Kristallisatoren aus der DE-AS-23 12 088 sowie der DE-AS-1 072 965 bekannt, bei denen ebenfalls die unberippten Rohrbögen im Behälter liegend angeordnet sind, so daß auch diese bekannten Kristallisatortypen die nachstehend beschriebenen Nachteile aufweisen.

Treten später während des Betriebs an den Schweißstellen zwischen den Rohrbögen und den angrenzenden Schenkelenden Undichtigkeiten auf, besteht die Möglichkeit, daß das Kühlmedium oder das Heizmedium in den Produktraum innerhalb des Behälters gelangen und das Produkt kontaminieren kann. Eine Reparatur undicht gewordener Schweißstellen ist in der Regel am Einsatzort eines Kristallisators nicht möglich, da dieser im Inneren nicht zugänglich ist. Ein solcher Kristallisator muß vielmehr aus dem Gewinnungsprozeß genommen und durch einen neuen Kristallisator ersetzt werden. Der undicht gewordene Kristallisator muß dann in einer hierfür geeigneten Werkstatt demontiert und repariert werden. Der zeitliche und personalmäßige Aufwand für die in diesem Zusammenhang rein handwerklich anfallenden Arbeiten ist erheblich. Der Gewinnungsprozeß wird während des Austauschs der Kristallisatoren längere Zeit reduziert.

Weitere Probleme ergeben sich im bekannten Fall durch die bei etwa 2000 Lastwechseln pro Jahr anfallenden Temperaturunterschiede zwischen der Beladung eines Kristallisators und dem Abschmelzvorgang.

So ist es z.B. aufgrund der relativ hohen Schmelztemperatur von PSA (131 °C) erforderlich, die Rippenrohre innenseitig mit einem Heizmedium zu beaufschlagen, das eine Temperatur von etwa 170 °C aufweist. Dieser Temperaturunterschied ist notwendig, um in wirtschaftlich befriedigender Weise das PSA von den Rippenrohren abzuschmelzen.

Andererseits muß zum Niederschlagen des PSA auf den Rippenrohren ein Kühlmedium mit einer Temperatur von etwa 50 °C durch die Rippenrohre gepumpt werden. Diese Wechselwirkung von Kühlung und Heizung mit Temperaturunterschieden bis zu 150° K führt zu einer extrem hohen Beanspruchung des Kristallisators, insbesondere zu erheblichen Spannungen in den Schweißnähten.

Während sich die Rippenrohre entsprechend der jeweiligen Temperaturbeanspruchung dehnen und kürzen können, trifft dies für die dazu parallel verlaufenden Seitenwände des Behälters nicht zu. Hierbei ist nämlich zu berücksichtigen, daß die Seitenwände von beiden Stirnwänden aus mit den Temperaturwechseln konfrontiert werden, da das Kühlmedium und das Heizmedium von beiden Stirnwänden her in die dort jeweils befestigten Rippenrohre gepumpt wird. Dabei treten besonders in den Eckbereichen zwischen den Stirnwänden und den Seitenwänden Spannungsspitzen auf, die zu Rißbildungen und damit zu Undichtigkeiten führen können.

Diesen Belastungen versuchte man dadurch beizukommen, daß auf die ebenen Seitenwände und Stirnwände Rohre geschweißt wurden, welche einen Anschluß an den Kreislauf für das Kühlmedium bzw. das Heizmedium erhielten, um so für einen Temperaturausgleich zu sorgen. Die Lösung führte zwar zu einer Verbesserung, befriedigte aber nicht.

Aufgrund des Sachverhalts, daß sowohl das Kühlmedium als auch das Heizmedium von beiden Stirnwänden des Behälters aus in die Rippenrohre gepumpt wird, erfolgt zwangsläufig eine gegenläufige Dehnung und Verkürzung der im benachbarten Bündel liegenden Rippenrohre. Dies kann dann zu einer Blockade der Axialbewegungen der Rippenrohre führen, wenn durch eine unkorrekte Betriebsweise des Kristallisators sich nicht schmelzbare Rückstände bilden, die zwei gegenläufig sich dehnende bzw. kürzende Rippenrohre gewissermaßen miteinander verspannen. Das Ergebnis einer derartigen Blockade ist, daß die Rippenrohre aus den Einschweißstellen der Stirnwände gerissen werden. Auch hierdurch kann das Kühlmedium oder das Heizmedium in den Produktraum innerhalb des Behälters treten und das Produkt kontaminieren oder sogar den gesamten Kristallisator unbrauchbar machen.

Der Erfindung liegt ausgehend vom Stand der Technik die **Aufgabe** zugrunde, einen Kristallisator zur Gewinnung von sublimationsfähigen Produkten aus der Gasphase zu schaffen, der einfacher aufgebaut, wirtschaftlicher montiert und bei undichten Schweißstellen direkt am Ort des Einsatzes kurzfristig repariert werden kann.

Die **Lösung** dieser Aufgabe besteht nach der Erfindung in den im kennzeichnenden Teil des Anspruchs 1 aufgeführten Merkmalen.

Danach werden die insbesondere unberippten freien Schenkelenden sämtlicher Rippenrohre nur noch an einer einzigen Stirnwand festgelegt, die dann zugleich den Rohrboden für eine Verteilerkammer bildet. Dies kann z.B. durch Einschweißen oder Einwalzen erfolgen. Demzufolge können sich alle Rippenrohre in Abhängigkeit von der inneren Beaufschlagung durch ein Kühlmedium oder ein Heizmedium immer nur in dieselbe Richtung verkürzen oder dehnen.

Dieser Sachverhalt macht sich mithin äußerst positiv auf die Verbindungsnähte zwischen den Stirnwänden und den sich jetzt mit den Rippenrohren dehnenden und zusammenziehenden Seitenwänden bemerkbar. Die Verbindungsnähte sind dadurch keinen Spannungsspitzen mehr unterworfen. Ein weiterer Vorteil besteht darin, daß die den Rohrbögen benachbarten Schenkelenden mit Gleitspiel in der den Rohrbögen benachbarten Stirnwand gelagert sind, so daß diese Lagerung keine negativen Auswirkungen auf die Standzeit des Kristallisators hat. Da die Rohrbögen in einer von dem Produktraum getrennten, zur Umgebung hin fluiddichten Ausdehnungskammer liegen, kann die gesamte Länge zwischen den beiden Stirnwänden genutzt werden, um die Rippenrohre mit Rippen zu bestücken. Die Anordnung der Rohrbögen in der Ausdehnungskammer hat darüber hinaus den Vorteil, daß die Schweißnähte zwischen den Rohrbögen und den angrenzenden Schenkelenden nach Entfernung der Rohrbogenhaube gut zugänglich sind und bei einer eventuellen Undichtigkeit problemlos an Ort und Stelle kurzfristig repariert werden können. Demzufolge entfällt der gesamte Aufwand, der bislang mit dem Austausch eines undicht gewordenen Kristallisators gegen einen neuen Kristallisator einhergeht.

Eine Blockade der Rippenrohre bei durch eine falsche Fahrweise gebildeten nicht schmelzbaren Rückständen kann auch nicht mehr eintreten, da alle Rippenrohre sich nur noch in dieselbe Richtung dehnen oder verkürzen können und keine gegenläufigen Bewegungen stattfinden.

Desweiteren ist es im Rahmen der Erfindung von Vorteil, daß die Zu- und Abführung des Kühlmediums bzw. des Heizmediums nur noch an einer Stirnwand des Kristallisators erfolgt. Der Aufwand zur Installation von Zu- und Abführleitungen wird hiermit erheblich gesenkt.

Ferner kann die Montage eines Kristallisators außerordentlich vereinfacht werden, da im Prinzip der stufenweise Aufbau einzelner Bündel und des Behälters entfallen kann. Die Rippenrohre werden insgesamt im Paket zwischen den Stirnwänden plaziert.

Entsprechend den weiterbildenden Merkmalen des Anspruchs 2 münden alle freien Schenkelenden der Rippenrohre in eine Verteilerkammer, die außenseitig der Stirnwand des Kristallisators befestigt, insbesondere angeschweißt, wird. Die in der Verteilerkammer ausgebildeten und von der Stirnwand als Rohrboden begrenzten Überleitungsräume sind einander so zugeordnet, daß das Kühlmedium oder Heizmedium in den untersten Überleitungsraum eingeleitet wird, von hier aus durch die Schenkel der an diesen Überleitungsraum und an den benachbarten nächst höheren Überleitungsraum angeschlossenen Rippenrohre strömt, von dem nächst höheren Überleitungsraum über die hieran angeschlossenen Schenkel der Rippenrohre in den darüber liegenden Überleitungsraum u.s.w. bis zum obersten Überleitungsraum gelangt und hier wieder austritt.

Die somit in einem einzigen Rippenrohrpaket liegenden Rippenrohre sind also hälftig, und zwar jedes zweite Rippenrohr, an einen unteren und an den nächst höheren Überleitungsraum angeschlossen. Die andere Hälfte der Rippenrohre ist dann an den nächst höheren Überleitungsraum sowie an den darüber liegenden Überleitungsraum angeschlossen.

Die Rohrstücke gemäß Anspruch 3 sind bevorzugt in die Stirnwand eingewalzt. Sie können aber auch eingeschweißt sein. Dabei ist das Spiel zwischen den Schenkelenden und den Rohrstücken so bemessen, daß eine gleitförmige Bewegung der Schenkelenden relativ zu den Rohrstücken zwar möglich ist, der Spalt zwischen den Schenkelenden und den Rohrstücken aber keinen merklichen Gasaustausch zwischen dem im Behälter strömenden und dem in der Ausdehnungskammer ruhenden Gas zuläßt. Die Länge der Rohrstücke kann etwa der doppelten Dicke der Stirnwand entsprechen. Somit finden die Schenkelenden ausreichenden Flächenkontakt, der einen vorzeitigen Reibungsverschleiß vermeidet.

Die Anordnung der Rippenrohre entsprechend den Merkmalen des Anspruchs 4 führt zu einer gewissermaßen stufenweise überlappenden Weiterleitung des Heizmediums oder Kühlmediums aus dem untersten Überleitungsraum in den obersten Überleitungsraum. Das heißt, jedes zweite der nebeneinander liegenden Rippenrohre ist an den untersten und an den benachbarten Überleitungsraum angeschlossen, während die dazwischen liegenden Rippenrohre an den dem untersten Überleitungsraum benachbarten Überleitungsraum und an den darüber liegenden Überleitungsraum angeschlossen sind. Die Anordnung auf Lücke führt zu einer dreieckförmigen Konfiguration der in der Vertikalen einander benachbarten Schenkel von drei übereinander liegenden horizontalen Schenkelreihen.

Da sich aufgrund der vorbeschriebenen Anordnung von Rippenrohren nicht besetzte Bereiche in der Nähe der Seitenwände ergeben, können diese Bereiche gemäß Anspruch 5 durch unberippte Rohre besetzt werden. Diese unberippten Rohre besitzen ebenfalls eine langgestreckte U-förmige Konfiguration. Ihre freien Schenkelenden sind an die Überleitungsräume angeschlossen, während die den Rohrbögen benachbarten Schenkelenden die der Verteilkammer gegenüberliegende Stirnwand gleitschlüssig durchsetzen. Die Rohrbögen liegen somit gleichfalls in der Ausdehnungskammer.

Da das Reaktionsgas mit dem darin enthaltenen Produkt nacheinander von oben nach unten die Zonen "reine Gaskühlung", "Gaskühlung mit Produktniederschlag" und "Nachkühlung" durchströmt, ist es erfindungsgemäß ferner von Vorteil, wenn die Lage und/oder die Ausbildung der berippten und ggf. auch der unberippten Rohre den jeweiligen Bedingungen angepaßt sind. Dies gilt insbesondere für den Produktabscheidebereich. Hier sollten entsprechend große Räume für die Ablagerung des Produkts zur Verfügung stehen, während engere Rohr- bzw. Rippenteilungen oder kleinere hydraulische Durchmesser in den Bereichen von Vorteil sind, in denen nur eine Gaskühlung stattfindet.

Dies kann entsprechend Anspruch 6 dadurch erfolgen, daß der Abstand der nebeneinander liegenden Rohre entsprechend der Gasströmrichtung zunächst von oben nach unten zunimmt, um dann im untersten Höhenbereich wieder abzunehmen. Dies kann gleichmäßig oder stufenweise erfolgen. Hierbei kann der Rippenabstand zwischen 4 mm und 20 mm variieren.

Denkbar ist es entsprechend Anspruch 7 aber auch, daß der hydraulische Durchmesser der nebeneinander liegenden Rohre zunächst von oben nach unten zunimmt und dann wieder abnimmt. Auch eine kombinative Ausgestaltung von Rohrabstand und Querschnittsgröße kann nach der Erfindung sinnvoll sein. Jeweils im mittleren Höhenbereich des Zentralbereichs ist der Abstand der Rohre und/oder der hydraulische Durchmesser am größten. Hier erfolgt die stärkste Produktabscheidung.

Obwohl die Erfindung es zuläßt, daß die Kernrohre der Rippenrohre und ggf. auch die unberippten Rohre einen ovalen Querschnitt aufweisen, ist es erfindungsgemäß vorteilhafter, daß die Rohre gemäß Anspruch 8 aus im Querschnitt runden Kernrohren mit umfangsseitig ggf. festgelegten wendelförmigen Rippen bestehen. Diese Rippen können am Außenumfang der Kernrohre festgelegt oder auch in wendelförmige Nuten der Kernrohre eingeklemmt sein. Vorstellbar sind auch Einzelrippen unterschiedlichster Konfiguration. Die Kernrohre können einen zylindrischen Durchmesser zwischen 20 mm und 50 mm bei entsprechenden Rippendurchmessern von 30 mm bis 100 mm aufweisen. Haben die Kernrohre z.B. einen Durchmesser von 25 mm, ist es zweckmäßig, den Innendurchmesser der Rohrstücke gemäß Anspruch 3 30 mm groß zu bemessen.

Da die Auflage der berippten und unberippten Rohre in den Stirnwänden, insbesondere bei Rohrlängen über 2 m, nicht ausreicht, um ihre Gewichte einschließlich des Gewichts des Produkts sowie des Kühl- oder Heizmediums aufzunehmen, ist es nach Anspruch 9 sinnvoll, daß alle Schenkel der Rohre von in den Seitenwänden des Behälters fluiddicht befestigten Trägerrohren an rippenfreien Stellen unterfangen sind. Diese Trägerrohre können durch die Seitenwände eingezogen und dort eingeschweißt sein.

Damit das Reaktionsgas an den rippenfreien Stellen der Rohre nicht von oben nach unten durchschlagen kann, ist es gemäß Anspruch 10 sinnvoll, daß die rippenfreien Stellen für die Trägerrohre von Höhenbereich zu Höhenbereich in der Horizontalen versetzt sind. Damit liegen nicht alle Trägerrohre in einer Vertikalebene übereinander und ungekühlte Gase werden vermieden.

Schließlich kann es entsprechend den Merkmalen des Anspruchs 11 noch von Vorteil sein, daß auf die Außenflächen der Gaseintrittshaube, der Seitenwände, der Produktsammelwanne und der die Ausdehnungskammer begrenzenden Rohrbogenhaube des Behälters Rohre zur Beaufschlagung mit dem Kühlmedium und mit dem Heizmedium verlegt sind. Hierbei kann es sich insbesondere um schlangenlinienförmig verlegte Rohre handeln, um die großen Innenflächen zu kühlen oder von Produktanhaftungen freischmelzen zu können.

Auch wenn im Vorstehenden von einer Strömung des Reaktionsgases von oben nach unten die Rede ist, so kann es durchaus denkbar sein, daß das Reaktionsgas den Behälter und damit die Rohre von unten nach oben passiert, während das Kühlmedium bzw. das Heizmedium dann im Kreuz-Gegenstrom von oben nach unten durch die Rohre fließt.

Die Herstellung des Kristallisators wird etwa wie folgt durchgeführt:
Zunächst werden alle horizontalen Schenkel der Rippenrohre und der unberippten Rohre unter Einsatz entsprechender Hilfskonstruktionen in die entsprechenden Öffnungen der Stirnwandabschnitte des mittleren Zentralbereichs des Behälters eingesetzt. Anschließend werden die freien Schenkelenden in dem von ihnen durchsetzten Stirnwandabschnitt festgelegt, insbesondere eingeschweißt. Danach werden die Rohrbögen an die Schenkel der Rippenrohre und der unberippten Rohre angeschweißt. Im Anschluß an diese Maßnahmen wird ein Seitenwandabschnitt des Zentralbereichs mit den beiden Stirnwandabschnitten verschweißt und dann werden die Trägerrohre eingezogen und mit diesem Seitenwandabschnitt verschweißt. Hierauf folgend können auch die anderen Enden der Trägerrohre in den anderen Seitenwandabschnitt eingefädelt, die Trägerrohre mit diesem Seitenwandabschnitt und der Seitenwandabschnitt mit den beiden Stirnwandabschnitten verschweißt werden. Sind diese Maßnahmen beendet, können die Verteilerkammer und die Rohrbogenhaube auf die Stirnwände geschweißt werden. Letztlich werden die Produktsammelwanne und die Gaseintrittshaube mit dem Zentralbereich verschweißt. Die umfangsseitigen Rohrschlangen werden zweckmäßig am Schluß auf die Außenflächen gesetzt und mit diesen verschweißt.

Die Erfindung ist nachfolgend anhand von in den Zeichnungen dargestellten Ausführungsbeispielen näher erläutert. Es zeigen:
- Fig. 1: einen vertikalen Längsschnitt durch einen Kristallisator zur Gewinnung von sublimationsfähigen Produkten aus der Gasphase;
- Fig. 2: eine Stirnansicht auf den Kristallisator gemäß dem Pfeil II der Fig. 1, teilweise im vertikalen Querschnitt entlang der Linien lla-lla, IIb-IIb;
- Fig. 3: eine Stirnansicht auf den Kristallisator gemäß dem Pfeil III der Fig. 1, teilweise im vertikalen Querschnitt entlang der Linie IIIa-IIIa;
- Fig. 4: eine Seitenansicht auf den Kristallisator entsprechend dem Pfeil IV der Fig. 2;
- Fig. 5: in vergrößerter Darstellung den Ausschnitt V der Fig. 1;
- Fig. 6: einen vertikalen abgeknickten Querschnitt im Bereich einer Seitenwand und einer Ausdehnungskammer eines Kristallisators entlang der Linie VI-VI der Fig. 5 und
- Fig. 7: in vergrößerter Darstellung den Ausschnitt VII der Fig. 2 gemäß einer weiteren Ausführungsform.

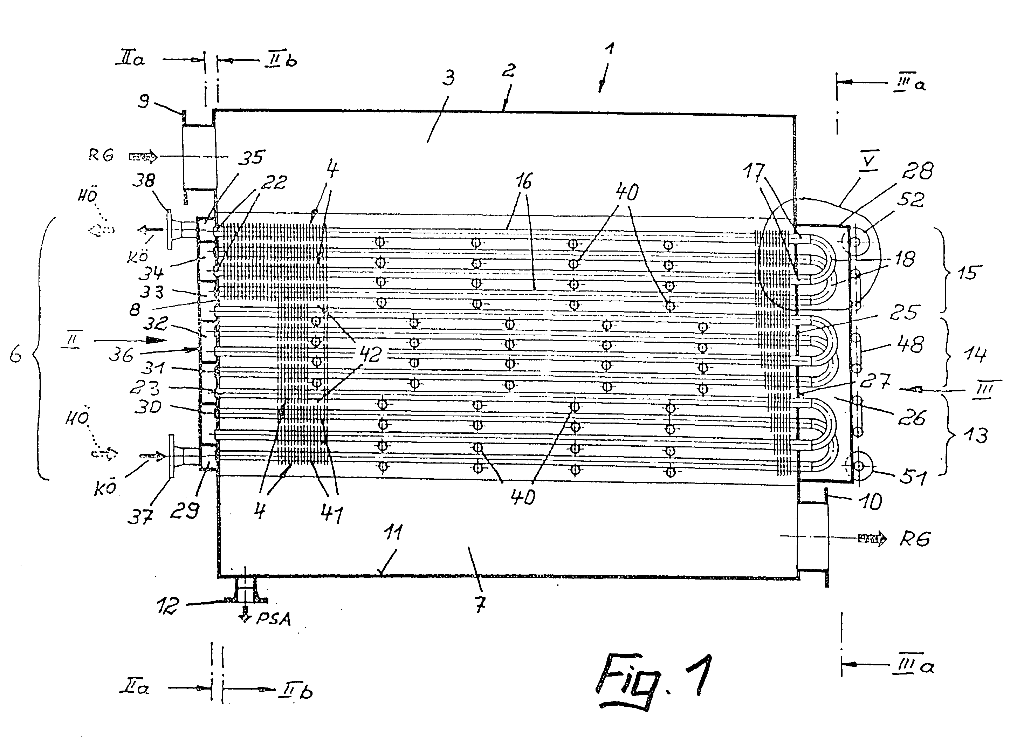

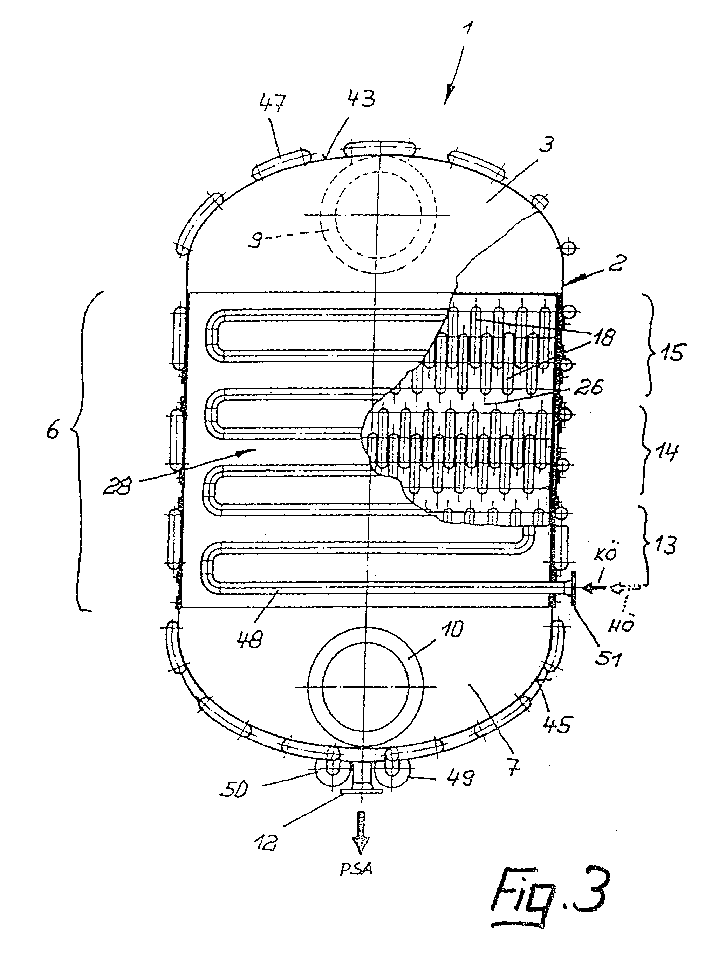

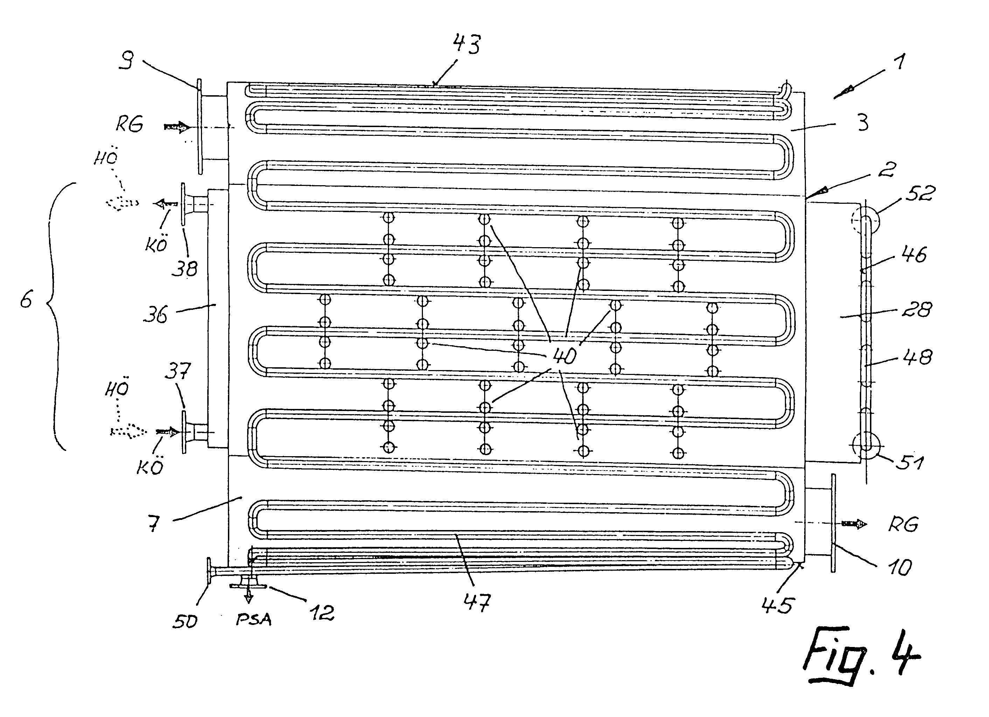

In den Fig. 1 bis 4 ist mit 1 ein Kristallisator zur Gewinnung von Phthalsäureanhydrid (PSA) aus einem Reaktionsgas RG bezeichnet.

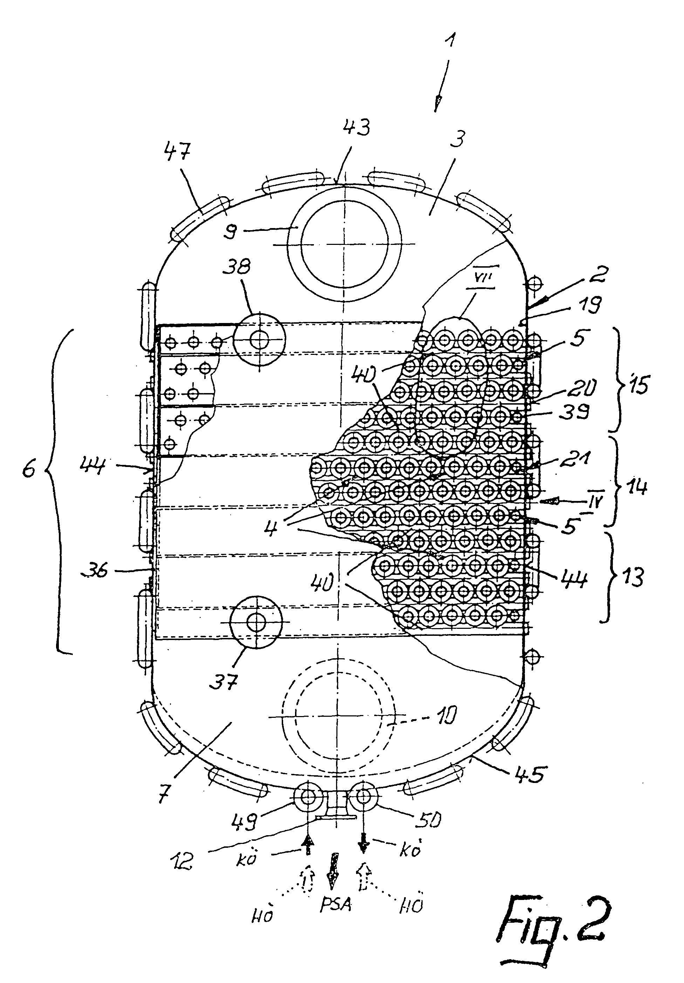

Der Kristallisator 1 umfaßt einen Behälter 2 mit einer Eintrittshaube 3 für das Reaktionsgas RG, mit einem von berippten Rohren 4 und unberippten Rohren 5 durchsetzten Zentralbereich 6 sowie mit einer unteren Sammelwanne 7 für das PSA (Fig. 1 und 2).

Die Eintrittshaube 3 ist mit einem Eintrittsflansch 9 für das Reaktionsgas RG versehen. Die Sammelwanne 7 besitzt an dem dem Eintrittsflansch 9 diagonal gegenüberliegenden Ende einen Austrittsflansch 10 für das Reaktionsgas RG. Desweiteren ist an der tiefsten Stelle der einen zur Stirnwand 8 geneigten Boden 11 aufweisenden Sammelwanne 7 ein Abzugsflansch 12 für PSA vorgesehen (Fig. 1).

Im Zentralbereich 6 des Behälters 2 sind übereinander liegend drei Höhenbereiche 13,14,15 mit berippten Rohren 4 und unberippten Rohren 5 langgestreckter U-förmiger Konfiguration vorgesehen. Jeder Höhenbereich 13,14,15 umfaßt mehrere nebeneinander liegende Rippenrohre 4 mit zwei horizontal verlaufenden berippten Schenkeln 16 und endseitigen, die nicht berippten Schenkelenden 17 der beiden Schenkel 16 verbindenden, ebenfalls nicht berippten Rohrbögen 18. Die Schenkel 16 jedes Rippenrohrs 4 erstrecken sich in einer vertikalen Ebene übereinander. Die Schenkel 16 benachbarter Rippenrohre 4 sind um etwa die Hälfte des Abstands der beiden Schenkel 16 eines Rippenrohrs 4 in der Höhe zueinander versetzt vorgesehen. Außerdem sind einander benachbarte Rippenrohre 4 auf Lücke angeordnet (Fig. 1 bis 3).

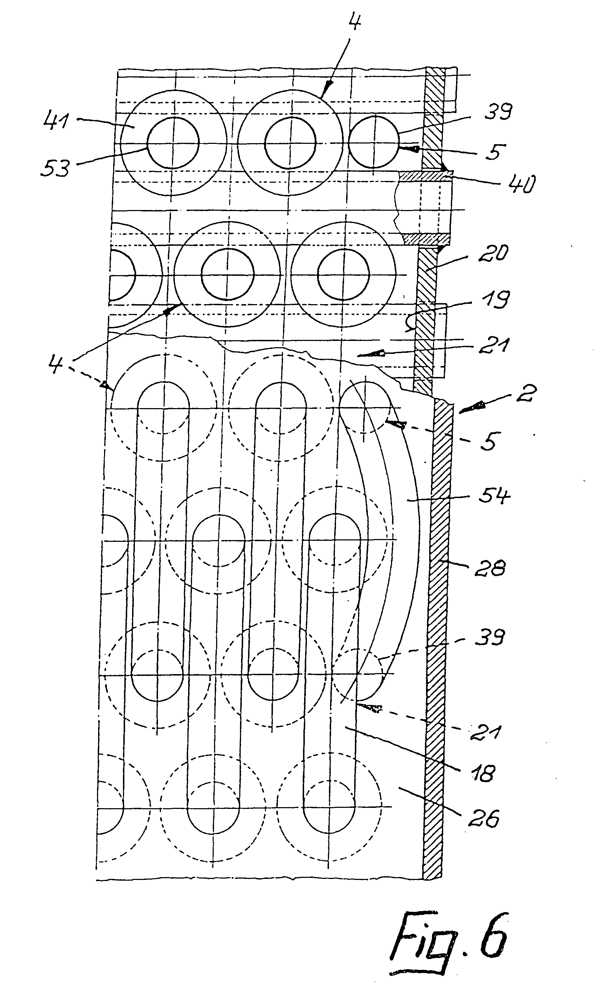

Durch diese Anordnung der Rippenrohre 4 in einem Höhenbereich 13,14,15 ergeben sich benachbart zu den Innenflächen 19 der Seitenwände 20 des Behälters 2 zwischen den in der Vertikalen einander benachbarten Schenkeln 16 der Rippenrohre 4 rohrfreie Bereiche 21, die, wie die Fig. 2 und 6 erkennen lassen, durch unberippte Rohre 5 langgestreckter U-förmiger Konfiguration durchsetzt werden.

Die unberippten freien Schenkelenden 22 aller Rohre 4,5 durchfassen Ausnehmungen 23 in der Stirnwand 8 des Behälters 2 und sind in dieser Stirnwand 8 durch Schweißen fluiddicht festgelegt (Fig. 1).

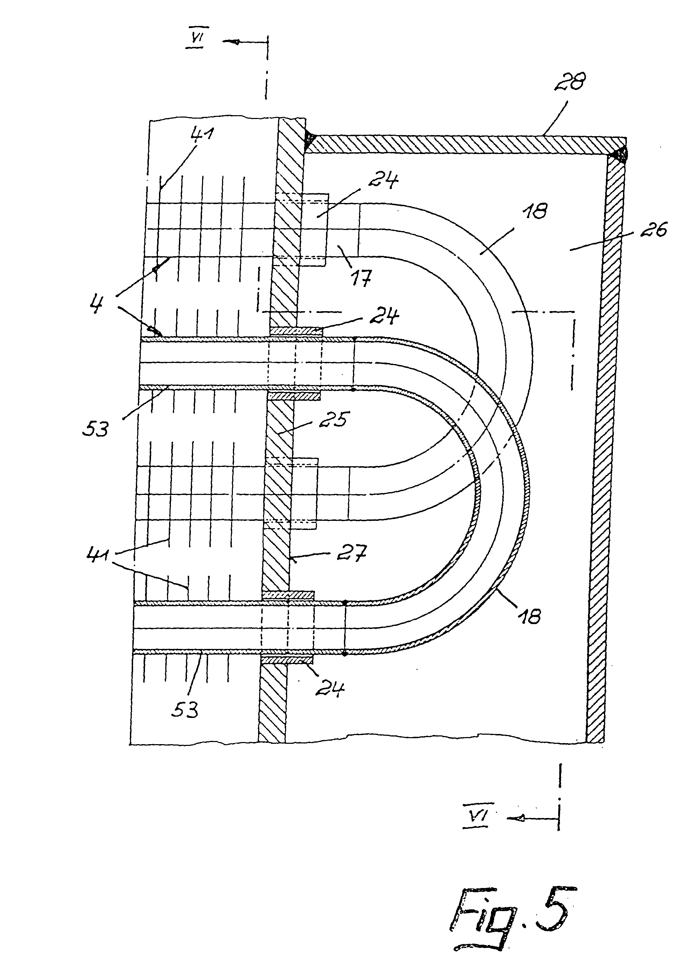

Die den Rohrbögen 18 benachbarten unberippten Schenkelenden 17 durchfassen gleitfähig Rohrstücke 24, die in der der Stirnwand 8 gegenüberliegenden Stirnwand 25 des Behälters 2 durch Schweißen festgelegt sind (Fig. 1 und 5). Die Länge der Rohrstücke 24 entspricht der doppelten Dicke der Stirnwand 25.

Die Rohrbögen 18 der Rippenrohre 4 sind mit den Schenkelenden 17 durch Schweißung verbunden (Fig. 5). Auch die Rohrbögen 54 der unberippten Rohre 5 sind mit den geraden Schenkeln 39 durch Schweißung verbunden. Diese Rohrbögen 54 verlaufen gemäß Fig. 6 bogenförmig, um mit den Rohrbögen 18 nicht in Kontakt zu gelangen.

Die Rohrbögen 18,54 aller Rohre 4,5 liegen in einer Ausdehnungskammer 26, welche durch die Außenfläche 27 der Stirnwand 25 und einer Rohrbogenhaube 28 gebildet ist. Die Rohrbogenhaube 28 ist auf die Außenfläche 27 der Stirnwand 25 fluiddicht geschweißt (Fig. 1,3,5 und 6).

Die freien Schenkelenden 22 der Rohre 4,5 münden in Überleitungsräume 29-35 einer Verteilerkammer 36 (Fig. 1 und 2). Diese ist auf die Stirnwand 8 geschweißt. Die Verteilerkammer 36 ist mit einem unteren Eintrittsflansch 37 und einem oberen Austrittsflansch 38 für ein Kühlöl KÖ bzw. ein Heizöl HÖ versehen.

Wie bei gemeinsamer Betrachtung der Fig. 1 bis 3 zu erkennen ist, sind die freien Schenkelenden 22 der Rippenrohre 4 so den Überleitungsräumen 29-35 zugeordnet, daß die unteren Schenkel 16 jedes zweiten Rippenrohrs 4 im untersten Höhenbereich 13 an den untersten Überleitungsraum 29 und die oberen Schenkel 16 an den nächst höheren Überleitungsraum 30 angeschlossen sind. Die unteren Schenkel 16 der daneben liegenden Rippenrohre 4 sind hingegen an den nächst höheren Überleitungsraum 30 und die oberen Schenkel 16 dieser Rippenrohre 4 an den darüber liegenden dritten Überleitungsraum 31 angeschlossen. Auf diese Weise gelangt das Kühlöl KÖ oder das Heizöl HÖ von dem untersten Überleitungsraum 29 über die daran angeschlossenen Rippenrohre 4 in den nächst höheren Überleitungsraum 30 und von diesem Überleitungsraum 30 über die daran angeschlossenen Rippenrohre 4 und jeden weiteren Überleitungsraum 31-35 sowie die damit verbundenen Rippenrohre 4 bis zum Austrittsflansch 38.

Die Fig. 2 und 6 zeigen, daß die nicht berippten Rohre 5 jeweils neben denjenigen Rippenrohren 4 liegen, deren Schenkel 16 zu den Innenflächen 19 der Seitenwände 20 weiter als die darüber und darunter liegenden Rippenrohre 4 distanziert sind. Die unberippten Rohre 5 im untersten Höhenbereich 13 sind an die Überleitungsräume 29 und 30, die im mittleren Höhenbereich 14 an die Überleitungsräume 31 und 32 und die Rohre 5 im oberen Höhenbereich 15 an die Überleitungsräume 33 und 34 angeschlossen.

Aus den Fig. 1,2,4 und 6 ist erkennbar, daß alle sich horizontal erstreckenden Schenkel 16 der Rippenrohre 4 und auch die Schenkel 39 der unberippten Rohre 5 im Abstand von Trägerrohren 40 unterfangen sind, welche in den Seitenwänden 20 des Behälters 2 fluiddicht befestigt sind. Dabei liegen die Trägerrohre 40 im obersten Höhenbereich 15 und im untersten Höhenbereich 13 in einer Vertikalebene übereinander, während die Trägerrohre 40 im dazwischen liegenden Höhenbereich 14 dazu in der Horizontalen versetzt sind. Hierdurch wird vermieden, daß das Reaktionsgas RG aus der Eintrittshaube 3 direkt in die Sammelwanne 7 durchschlagen kann. Die Fig. 1 zeigt hierbei, daß die Längenabschnitte 42 der Schenkel 16, welche von den Trägerrohren 40 unterfangen sind, keine Rippen 41 aufweisen.

Die berippten Rohre 4 besitzen im Innen- und Außenquerschnitt runde Kernrohre 53 (Fig. 5 und 6), die mit durchgehenden wendelförmigen Rippen 41 bestückt sind. Die Rippen 41 sind in nicht näher dargestellter Weise in Nuten befestigt, welche am Außenumfang der Kernrohre 53 eingearbeitet sind. Auch die unberippten Rohre 5 haben einen runden Innen- und Außenquerschnitt.

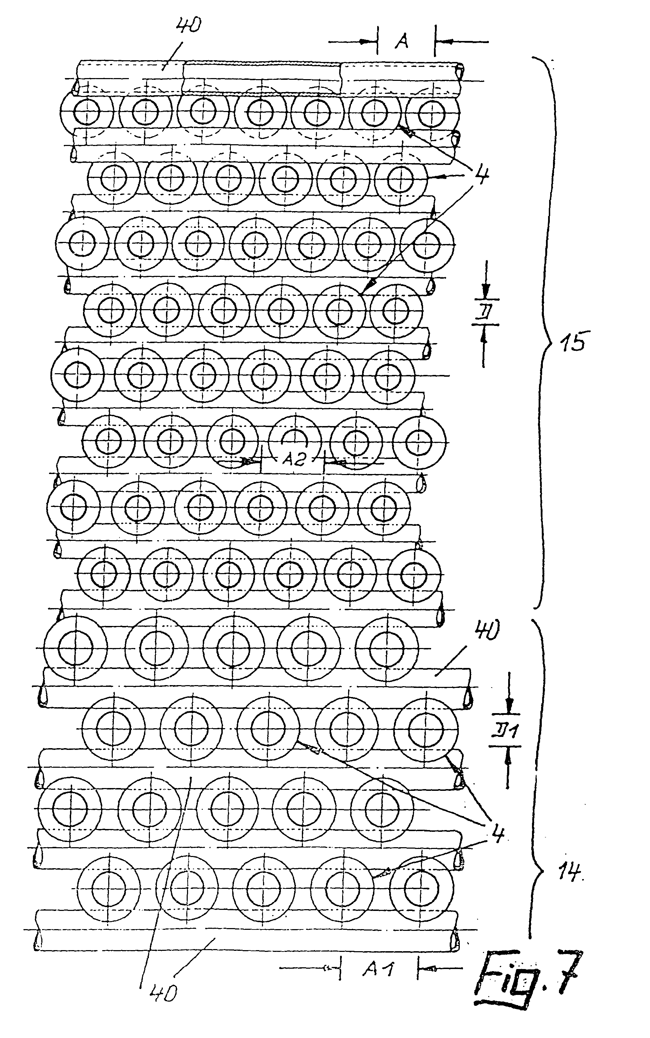

Die Ausführungsform der Fig. 7 läßt erkennen, daß der Seitenabstand A der im obersten Höhenbereich 15 einander benachbarten Rippenrohre 4 kleiner ist als der Seitenabstand A1 der Rippenrohre 4 im darunter befindlichen Höhenbereich 14. Der Seitenabstand A1 der im mittleren Höhenbereich 14 befindlichen Rippenrohre 4 ist wiederum größer als der Seitenabstand der im untersten nicht näher gezeichneten Höhenbereich 13 angeordneten Rippenrohre 4. Auf diese Art und Weise wird zwischen den Rippenrohren 4 des mittleren Höhenbereichs (Produktabscheidebereich) 14 mehr Raum zur Ablagerung von PSA geschaffen.

Der Seitenabstand A,A2 der Rippenrohre 4 im oberen Höhenbereich 15 und ggf. auch im unteren Höhenbereich 13 kann zusätzlich noch voneinander abweichen. Das heißt, der Seitenabstand A2 der dem mittleren Höhenbereich 14 benachbarten Rippenrohre 4 ist größer als der Seitenabstand A der zunächst von dem Reaktionsgas RG angeströmten Rippenrohre 4.

Wie die Fig. 7 ferner zeigt, können auch die hydraulischen Durchmesser D,D1 der Rippenrohre 4 in den Höhenbereichen 15,14 und 13 unterschiedlich groß bemessen sein. Das heißt, die hydraulischen Durchmesser D der Rippenrohre 4 im obersten Höhenbereich 15 sind kleiner als die hydraulischen Durchmesser D1 im mittleren Höhenbereich 14 und die hydraulischen Durchmesser D1 der Rippenrohre 4 im mittleren Höhenbereich 14 sind größer als die hydraulischen Durchmesser im unteren nicht näher dargestellten Höhenbereich 13 bemessen.

Die Abstände A,A2 und die hydraulischen Durchmesser D der Rippenrohre 4 im obersten Höhenbereich 15 können denen im untersten Höhenbereich 13 entsprechen. Sie können aber auch voneinander abweichen.

Bei gemeinsamer Betrachtung der Fig. 2 bis 4 ist festzustellen, daß auf den Außenflächen 43-46 der Eintrittshaube 3, der Seitenwände 20, der Sammelwanne 7 und der die Ausdehnungskammer 26 begrenzenden Rohrbogenhaube 28 des Behälters 2 schlangenlinienförmig verlegte unberippte Rohre 47,48 mit Anschlußflanschen 49,50,51,52 befestigt sind. Diese Rohre 47,48 können mit Kühlöl KÖ und Heizöl HÖ beaufschlagt werden.

Wird der Kristallisator 1 über den Eintrittsflansch 9 mit einem Reaktionsgas RG beaufschlagt, das PSA enthält, so werden die Rohre 4,5 über den Eintrittsflansch 37 an der Verteilerkammer 36 mit einem Kühlöl KÖ beaufschlagt, das eine Temperatur von etwa 50 °C besitzt. Das Kühlöl KÖ bewirkt, daß sich das PSA aus dem Reaktionsgas RG an den Oberflächen der Rippenrohre 4 und der unberippten Rohre 5 niederschlägt. Das somit von PSA entlastete Reaktionsgas RG verläßt den Kristallisator 1 über den Austrittsflansch 10. Auch die Rohre 47,48 können hierbei mit dem Kühlöl KÖ beaufschlagt werden.

Bei ausreichender Beladung der Rohre 4,5 mit PSA wird die Zufuhr von Reaktionsgas RG zum Kristallisator 1 unterbrochen. Gleichfalls erfolgt eine Unterbrechung der Zufuhr des Kühlöls KÖ zu den Rohren 4,5,47 und 48.

Die Rohre 4,5 und ggf. auch die Rohre 47,48 werden nunmehr mit einem Heizöl HÖ beaufschlagt, das eine Temperatur von etwa 170 °C besitzt. Aufgrund dieser Temperatur schmilzt das eine Schmelztemperatur von etwa 131 °C aufweisende PSA von den Rohren 4,5 und tropft in die Sammelwanne 7, von wo es über den Abzugsflansch 12 der Weiterbehandlung zugeführt wird.

Ist das PSA von den Rohren 4,5 abgeschmolzen, wird die Zufuhr von Heizöl HÖ wieder gestoppt, dafür Kühlöl KÖ in die Rohre 4,5,47 und 48 gepumpt und von neuem Reaktionsgas RG quer durch den Kristallisator 1 geleitet.

Bei dem Kühlöl KÖ und dem Heizöl HÖ kann es sich um ein und dasselbe Fluid handeln. Es wird dann nur entsprechend gekühlt oder aufgeheizt. Denkbar sind aber auch unterschiedliche Fluide.

### Bezugszeichenliste:

- 1: Kristallisator
- 2: Behälter v. 1
- 3: Eintrittshaube v. 2
- 4: berippte Rohre
- 5: unberippte Rohre
- 6: Zentralbereich v. 2
- 7: Sammelwanne v. 2
- 8: Stirnwand v. 2
- 9: Eintrittsflansch f. RG
- 10: Austrittsflansch f. RG
- 11: Boden v. 7
- 12: Abzugsflansch f. PSA
- 13: unterer Höhenbereich v. 6
- 14: mittlerer Höhenbereich v. 6
- 15: oberer Höhenbereich v. 6
- 16: Schenkel v. 4
- 17: Schenkelenden v. 4
- 18: Rohrbögen v. 4
- 19: Innenflächen v. 20
- 20: Seitenwände v. 2
- 21: rippenrohrfreie Bereiche
- 22: freie Schenkelenden v. 4
- 23: Ausnehmungen in 8 f. 22
- 24: Rohrstücke in 25
- 25: Stirnwand v. 2
- 26: Ausdehnungskammer
- 27: Außenfläche v. 25
- 28: Rohrbogenhaube
- 29: Überleitungsraum
- 30: Überleitungsraum
- 31: Überleitungsraum
- 32: Überleitungsraum
- 33: Überleitungsraum
- 34: Überleitungsraum
- 35: Überleitungsraum
- 36: Verteilerkammer
- 37: Eintrittsflansch f. KÖ u. HÖ
- 38: Austrittsflansch f. KÖ u. HÖ
- 39: Schenkel v. 5
- 40: Trägerrohre
- 41: Rippen auf 53
- 42: rippenfreie Stellen v. 16
- 43: Außenfläche v. 3
- 44: Außenfläche v. 20
- 45: Außenfläche v. 7
- 46: Außenfläche v. 28
- 47: Rohr auf 43-45
- 48: Rohr auf 46
- 49: Anschlußflansch v. 47
- 50: Anschlußflansch v. 47
- 51: Anschlußflansch v. 48
- 52: Anschlußflansch v. 48
- 53: Kernrohre v. 4
- 54: Rohrbögen v. 5

- A: Abstand v. 4 in 15
- A1: Abstand v. 4 in 14
- A2: Abstand v. 4 in 15
- D: hydr. Durchmesser v. 4 in 15
- D1: hydr. Durchmesser v. 4 in 14
- HÖ: Heizöl
- KÖ: Kühlöl
- PSA: Phthalsäureanhydrid
- RG: Reaktionsgas

## Patentansprüche

1. Kristallisator zur Gewinnung von sublimationsfähigen Produkten aus der Gasphase, welcher einen Behälter (2) in bündelartiger Anordnung mit ihren Schenkeln (16) horizontal durchsetzende, abschnittsweise unterstützte und mit ihren freien Schenkelenden (22) in der Stirnwand (8) des Behälter (2) festgelegte einzelne Rippenrohre (4) langgestreckter U-förmiger Konfiguration umfaßt, die bei innerer Beaufschlagung mit einem Kühlmedium (KÖ) von einem produktbeladenen Gas (RG) quer angeströmt sind und das dabei aus dem Gas (RG) abgeschiedene, auf den Rippenrohren (4) abgelagerte Produkt (PSA) bei innerer Beaufschlagung der Rippenrohre (4) mit einem Heizmedium (HÖ) von den Rippenrohren (4) abschmelzbar ist, wobei am Boden (11) des Behälters (2) ein Abzug (12) für das abgeschmolzene Produkt (PSA) vorgesehen ist,
**dadurch gekennzeichnet**,
daß alle Rippenrohre (4) mit ihren beiden Schenkelenden (22) in derselben Stirnwand (8) des Behälters (2) fluiddicht festgelegt sind und mit den an ihre Rohrbögen (18) angrenzenden unberippten Schenkelenden (17) die gegenüberliegende Stirnwand (25) des Behälters (2) relativ beweglich durchsetzent, wobei die Rohrbögen (18) in einer durch eine Rohrbogenhaube (28) und die benachbarte Stirnwand (25) begrenzten, zur Umgebung hin fluiddichten Ausdehnungskammer (26) liegen.

2. Kristallisator nach Anspruch 1, **dadurch gekennzeichnet**, daß die freien Schenkelenden (22) der Rippenrohre (4) in voneinander abgeschottete Überleitungsräume (29-35) münden, die Bestandteile einer außenseitig der Stirnwand (8) fluiddicht angeordneten Verteilerkammer (36) für das Kühlmedium (KÖ) und das Heizmedium (HÖ) bilden.

3. Kristallisator nach Anspruch 1 oder 2, **dadurch gekennzeichnet**, daß die den Rohrbögen (18) benachbarten Schenkelenden (17) in der die Ausdehnungskammer (26) begrenzenden Stirnwand (25) befestigte Rohrstücke (24) gleitfähig durchsetzen.

4. Kistallisator nach einem der Ansprüche 1 bis 3, **dadurch gekennzeichnet**, daß die jeweils nebeneinander liegenden Rippenrohre (4) um etwa die Hälfte des Abstandes ihrer sich horizontal übereinander erstreckenden Schenkel (16) in der Höhe zueinander versetzt angeordnet sind und ein jedes Rippenrohr (4) einer unteren Reihe zwischen zwei nebeneinander angeordneten Rippenrohren (4) der oberen Reihe angeordnet ist.

5. Kristallisator nach einem der Ansprüche 1 bis 4, **dadurch gekennzeichnet**, daß in die nicht von Rippenrohren (4) besetzten Bereiche (21) des Behälters (2) nahe der vertikalen Seitenwände (20) an die Überleitungsräume (29-34) angeschlossene unberippte Rohre (5) langgestreckter U-förmiger Konfiguration eingezogen sind.

6. Kristallisator nach einem der Ansprüche 1 bis 5, **dadurch gekennzeichnet**, daß der Abstand (A,A1,A2) der nebeneinander liegenden berippten und unberippten Rohre (4,5) im oberen und unteren Höhenbereich (15,13) des Zentralbereichs (6) des Behälters (2) kleiner als im mittleren Höhenbereich (14) bemessen ist.

7. Kristallisator nach einem der Ansprüche 1 bis 6, **dadurch gekennzeichnet**, daß der hydraulische Durchmesser (D,D1) der berippten und unberippten Rohre (4,5) im oberen und unteren Höhenbereich (15,13) des Zentralbereichs (6) des Behälters (2) kleiner als im mittleren Höhenbereich (14) bemessen ist.

8. Kristallisator nach einem der Ansprüche 1 bis 7, **dadurch gekennzeichnet**, daß die berippten Rohre (4) aus im Querschnitt runden Kernrohren (53) mit umfangsseitig festgelegten wendelförmigen Rippen (41) bestehen.

9. Kristallisator nach einem der Ansprüche 1 bis 8, **dadurch gekennzeichnet**, daß alle Schenkel (16,39) der berippten und unberippten Rohre (4,5) von in den Seitenwänden (20) des Behälters (2) fluiddicht befestigten Trägerrohren (40) an rippenfreien Stellen (42) unterfangen sind.

10. Kristallisator nach Anspruch 9, **dadurch gekennzeichnet**, daß die rippenfreien Stellen (42) für die Trägerrohre (40) von Höhenbereich (13 bzw. 14) zu Höhenbereich (14 bzw. 15) in der Horizontalen versetzt sind.

11. Kristallisator nach einem der Ansprüche 1 bis 10, **dadurch gekennzeichnet**, daß auf den Außenflächen (43-46) der Gaseintrittshaube (3), der Seitenwände (20), der Produktsammelwanne (7) und der die Ausdehnungskammer (26) begrenzenden Rohrbogenhaube (28) des Behälters (2) Rohre (47,48) zur Beaufschlagung mit dem Kühlmedium (KÖ) und dem Heizmedium (HÖ) verlegt sind.

## Claims

1. Crystallizer for obtaining products, which are capable of sublimation from the gas phase, which crystallizer comprises a vessel (2) which is in bundled arrangement with individual finned tubes (4) of elongate U-shaped configuration, which pass through in the horizontal direction by means of their limbs (16), are supported in sections, are fixed in the end wall (8) of the vessel (2) by means of their free limb ends (22) and, while being acted on internally by a cooling medium (KÖ), have a product-laden gas (RG) flowing transversely onto them, and the product (PSA) which has been separated out of the gas (RG) and has been deposited on the finned tubes (4), when the finned tubes (4) are internally exposed to a heating medium (HÖ), can be melted off the finned tubes (4), a tap (12) for the product (PSA) which has been melted off being provided at the bottom (11) of the vessel (2), **characterized in that**
all the finned tubes (4) are fixed in a fluid-tight manner, by means of their two limb ends (22), in the same end wall (8) of the vessel (2) and penetrate through the opposite end wall (25) of the vessel (2), in such a manner that they can move relative to one another, by means of the unfinned limb ends (17) which adjoin their tube bends (18), the tube bends (18) lying in an expansion chamber (26) which is delimited by a tube bend hood (28) and the adjacent end wall (25) and is fluid-tight with respect to the environment.

2. Crystallizer, according to Claim 1, **characterized in that** the free limb ends (22) of the finned tubes (4) open into transfer chambers (29-35) which are blocked off from one another and form components of a manifold chamber (36), which is arranged in a fluid-tight manner on the outside of the end wall (8), for the cooling medium (KÖ) and the heating medium (HÖ).

3. Crystallizer, according to Claim 1 or 2, **characterized in that** the limb ends (17) which adjoin the tube bends (18) pass in a slideable manner through tube sections (24) which are secured in the end wall (25) which delimits the expansion chamber (26).

4. Crystallizer, according to one of Claims 1 to 3, **characterized in that** the finned tubes (4) which lie next to one another are arranged vertically offset with respect to one another by approximately half the distance between their limbs (16) which extend horizontally above one another, and each finned tube (4) of a lower row is arranged between two finned tubes (4), arranged next to one another, of the upper row.

5. Crystallizer, according to one of Claims 1 to 4, **characterized in that** unfinned tubes (5) of elongate U-shaped configuration, which are connected to the transfer chambers (29-34), are pulled vertically into those regions (21) of the vessel (2) which are not occupied by finned tubes (4), in the vicinity of the vertical side walls (20).

6. Crystallizer, according to one of Claims 1 to 5, **characterized in that** the distance (A, A1, A2) between the adjacent finned and unfinned tubes (4, 5) in the upper and lower height regions (15, 13) of the central region (6) of the vessel (2) is less than in the middle height region (14).

7. Crystallizer, according to one of Claims 1 to 6, **characterized in that** the hydraulic diameter (D, D1) of the finned and unfinned tubes (4, 5) in the upper and lower height regions (15, 13) of the central region (6) of the vessel (2) is less than in the middle height region (14).

8. Crystallizer, according to one of Claims 1 to 7, **characterized in that** the finned tubes (4) comprise core tubes (53), which are round in cross section, with helical fins (41) fixed to the circumferential side.

9. Crystallizer, according to one of Claims 1 to 8, **characterized in that** all the limbs (16, 39) of the finned and unfinned tubes (4, 5) have support tubes (40), which are attached in a fluid-tight manner in the side walls (20) of the vessel (2), engaging beneath them at fin-free locations (42).

10. Crystallizer, according to Claim 9, **characterized in that** the fin-free locations (42) for the support tubes (40) are horizontally offset from height region (13 or 14) to height region (14 or 15).

11. Crystallizer, according to one of Claims 1 to 10, **characterized in that** tubes (47, 48) for applying the cooling medium (KÖ) and the heating medium (HÖ) are laid on the outer surfaces (43-46) of the gas inlet hood (3), of the side walls (20), of the product collection sump (7) and of the tube bend hood (28), which delimits the expansion chamber (26), of the vessel (2).

## Revendications

1. Cristallisoir d'extraction en phase gazeuse de produits sublimables, lequel comprend des tuyaux à ailettes (4) individuels allongés en U dont les branches (16) disposées en faisceau traversent horizontalement un récipient (2), qui sont soutenus par intervalles et sont fixés par leurs extrémités libres de branche (22) dans la paroi d'extrémité (8) du récipient (2) et qui, tout en étant alimentés intérieurement avec un fluide de refroidissement (KÖ) sont soumis à un écoulement transversal de gaz (RG) chargé de produit, le produit (PSA) extrait du gaz (RG) à cette occasion et qui s'est déposé sur les tuyaux à ailettes (4) étant séparable par fusion des tuyaux à ailettes (4) en alimentant intérieurement les tuyaux à ailettes (4) avec un fluide de chauffage (HÖ), un dispositif de soutirage (12) du produit fondu (PSA) étant prévu au fond (11) du récipient (2),
**caractérisé en ce que** tous les tuyaux à ailettes (4) sont fixés par leurs deux extrémités de branche (22) dans la même paroi d'extrémité (8) du récipient (2) de manière étanche aux fluides et en ce qu'ils traversent de manière relativement mobile la paroi d'extrémité (25) opposée du récipient (2) par leurs extrémités de branche (17), dépourvues d'ailettes, attenantes à leurs lyres (18), les lyres (18) se trouvant dans une chambre d'expansion (26) étanche aux fluides vis-à-vis du milieu environnant et délimitée par un capot de recouvrement des lyres (28) et par la paroi d'extrémité (25) voisine.

2. Cristallisoir selon la revendication 1, **caractérisé en ce que** les extrémités libres de branche (22) des tuyaux à ailettes (4) débouchent dans des espaces de mise en communication (29 à 35) rendus étanches les uns vis-à-vis des autres, lesquels font partie d'une chambre de distribution (36) disposée de manière étanche aux fluides sur la face extérieure de la paroi d'extrémité (8) et destinée au fluide de refroidissement (KÖ) et au fluide de chauffage (HÖ).

3. Cristallisoir selon la revendication 1 ou 2, **caractérisé en ce que** les extrémités de branche (17) voisines des lyres (18) traversent en pouvant glisser des tronçons de tube (24) fixés dans la paroi d'extrémité (25) délimitant la chambre d'expansion (26).

4. Cristallisoir selon l'une des revendications 1 à 3, **caractérisé en ce que** les tuyaux à ailettes (4) situés les uns à côté des autres sont à chaque fois disposés les uns par rapport aux autres en étant décalés en hauteur d'à peu près la moitié de l'écart entre leurs branches (16) qui s'étendent horizontalement l'une au-dessus de l'autre et en ce que chaque tuyau à ailettes (4) d'une rangée inférieure est disposé entre deux tuyaux à ailettes (4) placés l'un à côté de l'autre de la rangée supérieure.

5. Cristallisoir selon l'une des revendications 1 à 4, **caractérisé en ce que** des tuyaux (5) allongés en U, dépourvus d'ailettes et reliés aux espaces de mise en communication (29 à 34) sont insérés, à proximité des parois latérales verticales (20), dans les zones (21) du récipient (2) qui ne sont occupées par aucun tuyau à ailettes (4).

6. Cristallisoir selon l'une des revendications 1 à 5, **caractérisé en ce que** l'écart (A, A1, A2) entre les tuyaux pourvus ou dépourvus d'ailettes (4, 5) situés les uns à côté des autres est plus petit dans les tranches horizontales supérieure et inférieure (15, 13) de la partie centrale (6) du récipient (2) que dans la tranche horizontale médiane (14).

7. Cristallisoir selon l'une des revendications 1 à 6, **caractérisé en ce que** le diamètre hydraulique (D, D1) des tuyaux à ailettes (4) et des tuyaux dépourvus d'ailettes (5) est plus petit dans les tranches horizontales supérieure et inférieure (15, 13) de la partie centrale (6) du récipient (2) que dans la tranche horizontale médiane (14).

8. Cristallisoir selon l'une des revendications 1 à 7, **caractérisé en ce que** les tuyaux à ailettes (4) sont constitués de tubes centraux (53) à section transversale circulaire comportant des ailettes hélicoïdales (41) fixées sur leur pourtour.

9. Cristallisoir selon l'une des revendications 1 à 8, **caractérisé en ce que** toutes les branches (16, 39) des tuyaux à ailettes (4) et des tuyaux dépourvus d'ailettes (5) sont soutenus à des endroits dépourvus d'ailettes (42) par des tubes porteurs (40) fixés de manière étanche aux fluides dans les parois latérales (20) du récipient (2).

10. Cristallisoir selon la revendication 9, **caractérisé en ce que** les endroits dépourvus d'ailettes (42) destinés aux tubes porteurs (40) sont décalés horizontalement d'une tranche (13 ou 14) à une autre tranche (14 ou 15).

11. Cristallisoir selon l'une des revendications 1 à 10, **caractérisé en ce que** des tuyaux (47, 48) destinés à être alimentés en fluide de refroidissement (KÖ) ou en fluide de chauffage (HÖ) sont placés sur les surfaces extérieures (43 à 46) des éléments suivants du récipient (2) : dôme d'admission de gaz (3), parois latérales (20), bac collecteur de produit (7) et capot de recouvrement (28) des lyres qui délimite la chambre d'expansion (26).
